# EUROPEAN PATENT APPLICATION

(11) **EP 4 843 557 A2**
(43) Date of publication of application: **25.11.2026**
(21) Application number: 26178391.4
(22) Date of filing: 13.05.2026
(51) Int. Cl.: A61K 8/67, A61K 8/34, A61N 5/06, A61Q 19/08

(54) **PHOTOREACTIVE COMPOSITION, LIGHT IRRADIATION DEVICE AND SKIN CARE KIT COMPRISING THE SAME**

(30) Priority: 20.05.2025 KR 20250065299; 20.05.2025 KR 20250065313
(71) Applicant: Amorepacific Corporation, Seoul 04386 (KR)
(72) Inventor: KIM, Jihoon, Yongin-si, Gyeonggi-do 17074 (KR); LEE, Seungmin, Yongin-si, Gyeonggi-do 17074 (KR); CHOI, Joonho, Yongin-si Gyeonggi-do 17074 (KR); KIM, Jihyun, Yongin-si, Gyeonggi-do 17074 (KR); LEE, Eun Soo, Gyeonggi-do 17074 (KR); HONG, Sohyun, Yongin-si, Gyeonggi-do 17074 (KR); KWON, Minkyung, Yongin-si, Gyeonggi-do 17074 (KR); NAM, Jin, Yongin-si, Gyeonggi-do 17074 (KR); PARK, Wonseok, Yongin-si, Gyeonggi-do 17074 (KR); LEE, Jeongin, Yongin-si, Gyeonggi-do 17074 (KR)
(74) Representative: Germain Maureau

(57) **Abstract**

The present specification discloses a photoreactive composition exhibiting a synergistic effect on skin wrinkle improvement and elasticity enhancement when used together with light irradiation, a light irradiation device for skin care, a skin care kit comprising the same, and a skin beautifying method using the same. According to the present disclosure, an efficacy of being capable of providing an immediate skin wrinkle improvement effect is exhibited even with one-time application of a composition and light irradiation for a short time, and a durability in which the effect is maintained for one day or more to one week or more may be provided.

## Description

### BACKGROUND OF THE INVENTION

### Field of the Invention

In the present specification, a photoreactive composition, a light irradiation device, a skin beautifying kit comprising the same, and a skin beautifying method using the same are disclosed.

### Description of the Related Art

As interest in skin increases, a skin care industry is expanding to not only cosmetics and skin massage but also skin beautifying procedures and beautifying devices. Accordingly, as demand for a skin beautifying device capable of providing more immediate and higher beautifying efficacy to the skin than cosmetics increases, types of skin beautifying devices, such as a high frequency device, a low frequency device, a lifting device, a galvanic current device, an ultrasonic device, and the like using current, and an MTS method using a fine needle, and the like, for skin elasticity improvement, soothing, whitening, exfoliation, scalp care, and the like, in addition to a conventional vibrating puff for skin makeup and a vibrating cleanser, are also in a trend of being diversified.

However, as for a cosmetic composition used together with the skin beautifying device, a skin care set product is in a situation of being absent, which can provide a synergy effect when used together with the beautifying device, going beyond a medium for delivering an effect of the beautifying device.

### SUMMARY OF THE INVENTION

A problem to be solved by the present disclosure is to provide a composition capable of providing a synergistic effect on skin wrinkle improvement and elasticity enhancement when used together with a light irradiation device such as a skin beautifying device.

A problem to be solved by the present disclosure is to provide a light irradiation device capable of providing an effective skin improvement effect within a short time by using light of a specific wavelength band with a high light amount.

A problem to be solved by the present disclosure is to provide a composition capable of providing a synergistic effect on skin wrinkle improvement and elasticity enhancement when used together with a light irradiation device such as a skin beautifying device, and a skin care kit comprising the light irradiation device.

A problem to be solved by the present disclosure is to provide a skin beautifying method using a composition capable of providing a synergistic effect on skin wrinkle improvement and elasticity enhancement when used together with a skin beautifying device.

To solve the problem, an embodiment of the present disclosure provides a photoreactive composition, which comprises riboflavin 5'-phosphate or a salt thereof as an active ingredient, further comprises an absorption promoter of the riboflavin 5'-phosphate or the salt thereof, and is for being used in combination with light irradiation of a wavelength of 380 to 570 nm.

Also, an embodiment of the present disclosure provides a light irradiation device for skin care, comprising: a main body forming a housing in which a light irradiation part is installed; the light irradiation part provided in the main body and irradiating light in a wavelength band of 380 nm to 570 nm; and a power supply part supplying power to the light irradiation part, wherein a light amount of the light irradiation part is 10 to 70 mW/cm².

Also, an embodiment of the present disclosure provides a skin care kit comprising: the photoreactive composition; and a light irradiation device comprising a light irradiation part.

Also, an embodiment of the present disclosure provides a skin beautifying method comprising: applying the photoreactive composition to skin; and irradiating light in a wavelength band of 380 nm to 570 nm to the skin to which the composition is applied.

A composition according to embodiments of the present disclosure may exhibit a synergistic effect on skin wrinkle improvement and elasticity enhancement when used together with light irradiation in a visible light wavelength band. Accordingly, an efficacy of being capable of providing an immediate skin wrinkle improvement effect is exhibited even with one-time application of the composition and light irradiation for a short time, and a durability in which the effect is maintained for one day or more to one week or more may be provided.

### BRIEF DESCRIPTION OF THE DRAWINGS

FIG. 1 is a view showing an upper surface structure of a light irradiation device for skin care according to an embodiment of the present disclosure.
FIG. 2 is a functional block diagram of a light irradiation device for skin care according to an embodiment of the present disclosure.
FIG. 3 is a view showing a structure for each type of a light irradiation device for skin care according to various embodiments of the present disclosure.
FIG. 4 is a view showing a set configuration and a use process comprising a light irradiation device and a photoreactive material according to an embodiment of the present disclosure.
FIG. 5 is a flowchart of a skin care method using a light irradiation device of the present disclosure.
FIG. 6A is a view showing a facial photograph of a subject (untreated group) in a state before treatment with a composition and blue light according to an embodiment of the present disclosure in Test Example 1.
FIG. 6B is a view showing a facial photograph of a subject taken within 10 minutes after application of a composition and blue light irradiation according to an embodiment of the present disclosure in Test Example 1.
FIG. 7A is a view showing a fluorescence image for analyzing skin tissue density of a control group (untreated group) in Test Example 3.
FIG. 7B is a view showing a fluorescence image for analyzing skin tissue density of Comparative Example 1 (photoreactive composition alone-treated group) in Test Example 3.
FIG. 7C is a view showing a fluorescence image for analyzing skin tissue density of Comparative Example 2 (blue light alone-treated group) in Test Example 3.
FIG. 7D is a view showing a fluorescence image for analyzing skin tissue density of Example 1 (photoreactive composition + blue light treated group) in Test Example 3.

### DETAILED DESCRIPTION OF THE INVENTION

Hereinafter, exemplary embodiments of the present disclosure will be described in detail with reference to the accompanying drawings.

The embodiments of the present disclosure disclosed herein are exemplified for the purpose of description only, and the embodiments of the present disclosure may be implemented in various forms. Since the present disclosure may be subject to various modifications and take various forms, it should be understood to include all modifications, equivalents, or substitutes included in the technical teachings and scope of the present disclosure. Singular expressions include plural expressions unless clearly described as different meanings in the context. In the present application, the terms "comprises," "comprising," "includes," "including," "containing," "has," "having" or other variations thereof are inclusive and therefore specify the presence of stated features, integers, steps, operations, constituent elements, components (or parts), and/or combinations thereof, but do not exclude the presence or addition of one or more other features, integers, steps, operations, constituent elements, components (or parts), and/or combinations thereof.

An embodiment of the present disclosure provides a photoreactive composition, which comprises riboflavin 5'-phosphate or a salt thereof as an active ingredient, further comprises an absorption promoter of the riboflavin 5'-phosphate or the salt thereof, and is for being used in combination with light irradiation of a wavelength of 380 to 570 nm.

In the present disclosure, the 'photoreactivity' means a property of promoting a biochemical reaction and a photochemical reaction within skin tissue in response to light in a wavelength band of 380 to 570 nm.

In an embodiment, the combined use of the composition and light irradiation may include both irradiating light to a skin application site within a short time after applying the composition to skin or irradiating light to the skin application site simultaneously with applying the composition to skin.

In the present disclosure, the riboflavin 5'-phosphate is an active form of B2 and mainly converted into a flavin protein in the body to act as a cofactor for various enzymes, and when used for skin, it may provide a useful efficacy through an antioxidant action. However, a synergistic effect in which efficacy for skin is enhanced when light is irradiated after applying the riboflavin 5'-phosphate or the salt thereof to skin has not been reported.

In an embodiment, the salt of riboflavin 5'-phosphate is not limited as long as it is a cosmetic composition or a pharmaceutically acceptable salt, and for example, an acid addition salt, a base addition salt, and an amino acid salt may be mentioned. For example, the salt of the riboflavin 5'-phosphate comprises inorganic acid salts such as hydrochloride, hydrobromide, sulfate, hydroiodide, nitrate, phosphate, and the like; organic acid salts such as citrate, oxalate, acetate, formate, propionate, benzoate, trifluoroacetate, maleate, tartrate, methanesulfonate, benzenesulfonate, para-toluenesulfonate, and the like; inorganic base salts such as sodium salt, potassium salt, calcium salt, magnesium salt, copper salt, zinc salt, aluminum salt, ammonium salt, and the like; organic base salts such as triethylammonium salt, triethanolammonium salt, pyridinium salt, diisopropylammonium salt, and the like; and amino acid salts such as lysine salt, arginine salt, histidine salt, aspartate, glutamate, and the like. In an embodiment, the salt of the riboflavin 5'-phosphate may be a water-soluble salt, and specifically, may be an inorganic base salt. More specifically, the salt of the riboflavin 5'-phosphate may be an alkali metal salt such as a sodium salt or a potassium salt of riboflavin 5'-phosphate.

In an embodiment, the composition may comprise the riboflavin 5'-phosphate or the salt thereof in an amount of 0.0001 to 10 wt% based on a total weight of the composition. Specifically, a content of the riboflavin 5'-phosphate or the salt thereof may be 0.0001 wt% or more, 0.001 wt% or more, 0.01 wt% or more, 0.05 wt% or more, 0.1 wt% or more, 0.5 wt% or more, 1.0 wt% or more, 1.5 wt% or more, 2 wt% or more, 2.5 wt% or more, 3 wt% or more, 3.5 wt% or more, 4 wt% or more, 4.5 wt% or more, 5 wt% or more, 5.5 wt% or more, 6 wt% or more, 6.5 wt% or more, 7 wt% or more, 7.5 wt% or more, 8 wt% or more, 8.5 wt% or more, 9 wt% or more, or 9.5 wt% or more, and may be 10 wt% or less, 9.5 wt% or less, 9 wt% or less, 8.5 wt% or less, 8 wt% or less, 7.5 wt% or less, 7 wt% or less, 6.5 wt% or less, 6 wt% or less, 5.5 wt% or less, 5 wt% or less, 4.5 wt% or less, 4 wt% or less, 3.5 wt% or less, 3 wt% or less, 2.5 wt% or less, 2 wt% or less, 1.5 wt% or less, 1 wt% or less, 0.5 wt% or less, 0.1 wt% or less, 0.05 wt% or less, 0.01 wt% or less, or 0.001 wt% or less, based on the total weight of the composition. When the content is less than 0.0001 wt%, a desired useful effect may appear insignificantly, and when it exceeds 10 wt%, skin irritation may appear.

In an embodiment, the absorption promoter is an additive for promoting skin absorption of the riboflavin 5'-phosphate or the salt thereof, and for example, propylene glycol, pentylene glycol, hexanediol, bis-ethoxydiglycol cyclohexane 1,4-dicarboxylate, diethoxyethyl succinate, dimethyl isosorbide, polyglyceryl-6 caprylate, and polyglyceryl-4 caprate may be mentioned. In an embodiment, the absorption promoter may be comprised in an amount of 1 to 40 wt% based on the total weight of the composition. Specifically, the absorption promoter may be comprised in an amount of 1 wt% or more, 5 wt% or more, 10 wt% or more, 15 wt% or more, 20 wt% or more, 25 wt% or more, 30 wt% or more, or 35 wt% or more, and may be comprised in an amount of 40 wt% or less, 35 wt% or less, 30 wt% or less, 25 wt% or less, 20 wt% or less, 15 wt% or less, 10 wt% or less, or 5 wt% or less, based on the total weight of the composition.

In an embodiment, light irradiated to skin along with the use of the composition may be visible light of a blue light series. Specifically, a wavelength range of the irradiated light may be 380 to 570 nm. More specifically, the wavelength range of the irradiated light may be 380 nm or more, 400 nm or more, 420 nm or more, 440 nm or more, 460 nm or more, 480 nm or more, 500 nm or more, 520 nm or more, 540 nm or more, or 560 nm or more, and may be 570 nm or less, 550 nm or less, 530 nm or less, 510 nm or less, 490 nm or less, 470 nm or less, 450 nm or less, 430 nm or less, 410 nm or less, or 390 nm or less. In an embodiment, when visible light of the blue light series is irradiated along with the use of the composition, a density of protein present in skin cell tissue increases due to a synergy with riboflavin 5'-phosphate or the salt thereof, thereby being capable of providing an immediate efficacy for skin wrinkle relief.

In an embodiment, the composition may be for being used in a skin beautifying method comprising one or more of: a light amount of the irradiated light being 1 to 100 mW/cm²; a light irradiation distance with respect to the skin being 0.1 to 30 cm; and a light irradiation time being 1 to 60 minutes.

In an embodiment, the light amount of the irradiated light may be 1 to 100 mW/cm². Specifically, the light amount may be 1 mW/cm² or more, 10 mW/cm² or more, 20 mW/cm² or more, 30 mW/cm² or more, 40 mW/cm² or more, 50 mW/cm² or more, 60 mW/cm² or more, 70 mW/cm² or more, 80 mW/cm² or more, or 90 mW/cm² or more, and may be 100 mW/cm² or less, 90 mW/cm² or less, 80 mW/cm² or less, 70 mW/cm² or less, 60 mW/cm² or less, 50 mW/cm² or less, 40 mW/cm² or less, 30 mW/cm² or less, 20 mW/cm² or less, or 10 mW/cm² or less.

In an embodiment, the light irradiation distance with respect to the skin may be 0.1 to 30 cm, and the distance may be appropriately adjusted according to a form, a light amount, or a method of use of the beautifying device. Specifically, the light irradiation distance with respect to the skin may be 0.1 cm or more, 1 cm or more, 2 cm or more, 3 cm or more, 4 cm or more, 5 cm or more, 6 cm or more, 7 cm or more, 8 cm or more, 9 cm or more, 10 cm or more, 15 cm or more, 20 cm or more, or 25 cm or more, and may be 30 cm or less, 25 cm or less, 20 cm or less, 15 cm or less, 10 cm or less, 9 cm or less, 8 cm or less, 7 cm or less, 6 cm or less, 5 cm or less, 4 cm or less, 3 cm or less, 2 cm or less, or 1 cm or less. When the light irradiation distance becomes too far, a light amount reaching the skin is small, so an effect may decrease.

As an embodiment, the light irradiation time may be 1 to 60 minutes, and this may be appropriately adjusted according to a desired effect or convenience of use. Specifically, the light irradiation time may be 1 minute or more, 5 minutes or more, 10 minutes or more, 15 minutes or more, 20 minutes or more, 25 minutes or more, 30 minutes or more, 35 minutes or more, 40 minutes or more, 45 minutes or more, 50 minutes or more, or 55 minutes or more, and may be 60 minutes or less, 55 minutes or less, 50 minutes or less, 45 minutes or less, 40 minutes or less, 35 minutes or less, 30 minutes or less, 25 minutes or less, 20 minutes or less, 15 minutes or less, 10 minutes or less, or 5 minutes or less.

In an embodiment, the skin beautifying method may identically comprise those described in a skin beautifying method to be described later.

In the embodiment, the composition may be a cosmetic composition.

In the embodiment, the composition may be for prevention of skin wrinkle generation or wrinkle improvement. In an embodiment, the composition may be for prevention of skin elasticity decrease or elasticity improvement. In an embodiment, the composition may be for improvement of nasolabial folds.

In the present disclosure, the nasolabial fold is also called a Beopryung line, and means a skin wrinkle of a face generated from an ala nasi to an oral angle (corner of the mouth).

Another embodiment of the present disclosure may provide a method for prevention of skin wrinkle generation or wrinkle improvement, or prevention of skin elasticity decrease or elasticity improvement, comprising the steps of: applying an effective amount of the riboflavin 5'-phosphate or the salt thereof and the absorption promoter to skin of a subject in need thereof; and irradiating light of a wavelength of 380 to 570 nm to the skin of the subject. Another embodiment may provide a use of the riboflavin 5'-phosphate or the salt thereof and the absorption promoter for manufacturing a skin photoreactive composition for prevention of skin wrinkle generation or wrinkle improvement, or prevention of skin elasticity decrease or elasticity improvement, and for irradiating light of a wavelength of 380 to 570 nm. Another embodiment may provide the riboflavin 5'-phosphate or the salt thereof and the absorption promoter for being used together with light irradiation of a wavelength of 380 to 570 nm in a composition for prevention of skin wrinkle generation or wrinkle improvement, or prevention of skin elasticity decrease or elasticity improvement.

In an embodiment, an appearance of the composition may contain a cosmetologically or dermatologically acceptable medium or base. For example, it may be provided in the form of a solution, a gel, a solid, a pasty anhydrous product, an emulsion obtained by dispersing an oil phase in an aqueous phase or an emulsion obtained by dispersing an aqueous phase in an oil phase, a suspension, a microemulsion, a microcapsule, a microgranule, or an ionic (liposome) and non-ionic vesicle dispersant. These compositions may be manufactured according to a conventional method in the relevant field.

In an embodiment, the cosmetic composition may be formulated into cosmetics such as an ampoule, a cream, a softening cosmetic water, an astringent cosmetic water, a nourishing cosmetic water, a nourishing cream, a gel cream, a massage cream, an essence, an eye cream, an eye essence, a cleansing cream, a cleansing foam, a cleansing water, a pack (or facial mask), a body lotion, a body cream, a body oil, and a body essence. When a formulation according to an embodiment of the present disclosure is a paste, a cream, or a gel, animal fiber, vegetable fiber, wax, paraffin, starch, tragacanth, cellulose derivatives, polyethylene glycol, silicone, bentonite, silica, talc, or zinc oxide, etc. may be used as a carrier component. When a formulation according to an embodiment of the present disclosure is a spray, lactose, talc, silica, aluminum hydroxide, calcium silicate, or polyamide powder may be used as a carrier component, and additionally, a propellant such as chlorofluorohydrocarbon, propane/butane, or dimethyl ether may be comprised. When a formulation according to an embodiment of the present disclosure is a solution or an emulsion, a solvent, a solvating agent, or an emulsifying agent is used as a carrier component, and there are, for example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, or fatty acid ester of sorbitan. When a formulation according to an embodiment of the present disclosure is a suspension, a liquid diluent such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, or tragacanth, etc. may be used as a carrier component. When a formulation according to an embodiment of the present disclosure is a surfactant-containing cleansing, aliphatic alcohol sulfate, aliphatic alcohol ether sulfate, sulfosuccinic acid monoester, isethionate, imidazolinium derivatives, methyl taurate, sarcosinate, fatty acid amide ether sulfate, alkylamido betaine, aliphatic alcohol, fatty acid glyceride, fatty acid diethanolamide, vegetable oil, lanolin derivatives, or ethoxylated glycerol fatty acid ester, etc. may be used as a carrier component.

In the cosmetic composition according to an embodiment of the present disclosure, functional additives and components comprised in general cosmetic compositions may be additionally comprised. The functional additives may comprise a component selected from the group consisting of water-soluble vitamins, oil-soluble vitamins, polymer peptides, polymer polysaccharides, sphingolipids, and seaweed extract. As blending components comprised other than these, oil and fat components, a moisturizer, an emollient, a neutralizing agent, a surfactant, organic and inorganic pigments, organic powder, an ultraviolet absorber, an antiseptic, a germicide, an antioxidant, plant extracts, a pH adjuster, alcohol, a colorant, a fragrance, a blood circulation promoter, a cooling agent, an antiperspirant, purified water, etc. may be mentioned.

In an embodiment, the composition may be a composition for external application to skin. As an embodiment, the composition for external application to skin may be applied directly by hand or may be applied by using various application means such as a spatula, a brush, and a puff.

In an embodiment, the composition may be a pharmaceutical composition. When a composition according to an embodiment of the present disclosure is applied to a medicine, it may be formulated as a parenteral administration agent in a semi-solid or liquid form by adding an inorganic or organic carrier commonly used for an active ingredient used in an embodiment of the present disclosure. As an embodiment, the pharmaceutical composition may be a parenteral administration agent, more specifically, an agent for external application to skin. For example, the agent for external application to skin may be a formulation of an ointment, a toner, a lotion, a gel, a cream, a spray, a suspension, an emulsion, a patch, and the like, but is not limited thereto. The composition according to one embodiment of the present disclosure may easily formulate the active ingredient by being carried out according to a conventional method widely known in the art, and in this case, a surfactant, an excipient, a coloring agent, a fragrance, a preservative, a stabilizer, a buffer, a suspending agent, and other commonly used auxiliary agents may be appropriately used. A dosage amount of the active ingredient of the pharmaceutical composition according to an embodiment of the present disclosure will depend on an age, sex, weight, pathologic state and severity of a subject to be administered, a route of administration, or judgment of a prescriber. Determination of an appropriate dose based on these factors is within the level of those skilled in the art, and a daily administration dose thereof may be, for example, 0.1 mg/kg/day to 100 mg/kg/day, more specifically 5 mg/kg/day to 50 mg/kg/day, but is not limited thereto. In the embodiment above, the composition may be for preventing or treating skin diseases occurring due to decrease in skin wrinkles or elasticity. In an embodiment, the composition may be for preventing or treating skin diseases caused by decrease in skin barrier function.

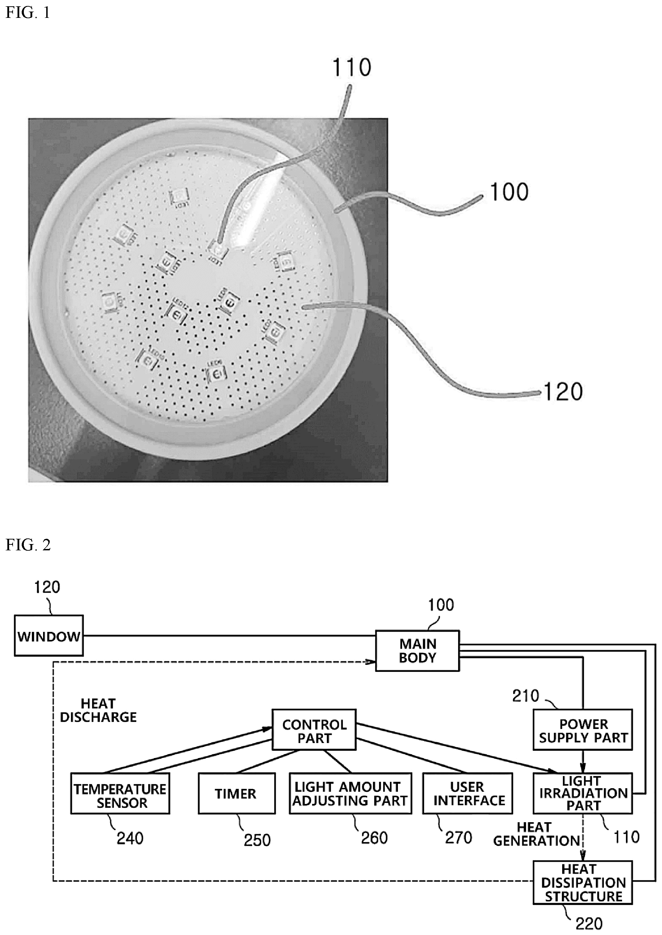

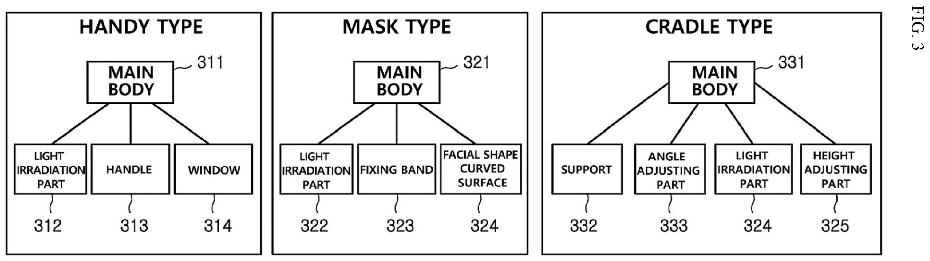

Hereinafter, embodiments will be described centering on basic components (or constituent elements) of the present disclosure shown in FIG. 1. Detailed descriptions of additional components such as a heat dissipation structure, a temperature sensor, and a control part, and embodiments of various forms such as a handy type, a mask type, and a cradle type, will be provided in FIG. 2 and FIG. 3 to be described later.

### Embodiment 1: Basic Configuration of Main Body, Light Irradiation Part, and Window

As shown in FIG. 1, a light irradiation device according to an embodiment of the present disclosure includes a circular main body 100, a light irradiation part 110 comprising a plurality of LEDs, and a transparent window 120.

The main body 100 serves to support and protect the light irradiation part 110, and in the embodiment of FIG. 1, it was implemented in a circular structure with a diameter of about 7 cm. The main body 100 is mainly composed of ABS resin having excellent heat resistance and durability, and may include an internal frame of a metal material with high thermal conductivity as necessary. An outside of the main body 100 is designed in a form that a user can comfortably hold, and a non-slip treatment is applied to a surface, so that stable use is possible.

The light irradiation part 110 is installed inside the main body 100, and as can be seen in FIG. 1, a total of 12 LEDs are arranged in a specific pattern. The LEDs are designed such that 4 are arranged in a center part and 8 are arranged in a peripheral part to enable uniform light irradiation. A wavelength of light generated by each LED may be 380 nm to 570 nm, and as an example, blue light in a wavelength band of 400 nm to 480 nm may be generated. In this case, it is known that the blue light is a wavelength band effective for skin elasticity improvement and wrinkle relief.

The LEDs of the light irradiation part 110 are of high output specifications, and a total light amount was set to 40 mW/cm². This is a considerably higher level than a general LED beautifying device (5 mW/cm² to 15 mW/cm²), and an effective skin improvement effect can be provided within a short time (15 minutes). The LED arrangement is optimized so that light can be uniformly irradiated to skin, and a beam angle of each LED is designed to be 120°, so that a wide range of skin areas can be covered. A temperature of the light irradiation part may be between 30 to 40°C upon use for 1 minute, and between 35 to 60°C upon use for 2 minutes.

The window 120 is located at a lower end part of the main body 100 and is installed between the light irradiation part 110 and the skin. The window 120 is manufactured of a transparent acrylic material with a thickness of about 2 mm, and is designed so that light generated from the LEDs can be delivered to the skin with minimum loss. A diameter of the window 120 is about 6 cm, and as can be seen in FIG. 1, a plurality of small holes (diameter of about 1 mm) are uniformly distributed on a surface. These holes serve to help heat generated during use of the device to be effectively discharged while maintaining light delivery efficiency.

An arrangement pattern of the holes is designed to achieve harmony with the LED arrangement, and an interval between each hole is uniformly maintained at about 3 mm. The size and interval of the holes are optimized within a range that does not harm structural stability of the window 120, and through this, heat generated from the LEDs is effectively dispersed and at the same time, light transmittance is also maintained high.

The window 120 not only enables hygienic use by preventing direct contact between the LEDs and the skin, but also performs a role of protecting the skin from heat generated from the high output LEDs. Also, the window 120 is designed to be easily separated and washed, so that hygienic device management is possible.

The configurations of the main body 100, the light irradiation part 110, and the window 120 described above show the most basic embodiment of the present disclosure, and thereafter, additional components and embodiments of various forms will be described in detail in FIG. 2 and FIG. 3.

### Embodiment 2: Additional Configuration

FIG. 2 is a view showing a functional block diagram of a light irradiation device for skin care according to an embodiment of the present disclosure.

As shown in FIG. 2, the light irradiation device of the present disclosure further includes a power supply part 210, a heat dissipation structure 220, a control part 230, a temperature sensor 240, a timer 250, a light amount adjusting part 260, and a user interface 270, in addition to the main body 100, the light irradiation part 110, and the window 120 described in FIG. 1.

The power supply part 210 serves to supply power to each component of the device including the light irradiation part 110 and the control part 230. The power supply part 210 may be implemented in a form of a rechargeable lithium-ion battery or a power cable. In the case of being implemented as a battery, a lithium-ion battery of 1,000 mAh to 2,000 mAh capacity may be used, and it is designed to enable continuous use for about 4 minutes to 60 minutes upon full charge. In the case of being implemented in a form of a power cable, stable power may be supplied through a universal adapter of an input voltage of 100 V to 240 V.

The heat dissipation structure 220 is a structure for effectively discharging heat generated during operation of the high output LEDs, and is installed inside the main body 100. The heat dissipation structure 220 may be configured by including a heat sink manufactured of an aluminum alloy, a heat dissipation fin, a thermally conductive pad, and the like. The heat sink is located directly below the LEDs to directly absorb heat generated from the LEDs, and the heat dissipation fin serves to effectively discharge the absorbed heat to the outside of the device. In the present embodiment, efficient heat dispersion is achieved by using an aluminum alloy (thermal conductivity: about 200 W/m·K) with high thermal conductivity.

The control part 230 includes a microcontroller controlling overall operations of the device, and is connected to the temperature sensor 240, the timer 250, the light amount adjusting part 260, the user interface 270, and the like, to integrally manage functions of the device. The control part 230 controls operation of the light irradiation part 110 based on temperature data received from the temperature sensor 240, manages operating time of the device through the timer 250, and adjusts output of the LEDs through the light amount adjusting part 260.

The temperature sensor 240 is installed near the light irradiation part 110 to measure a temperature of the LEDs in real time. When the measured temperature exceeds a set threshold (e.g., 60°C), a safety mechanism is operated to limit or stop the operation of the LEDs by delivering this information to the control part 230. In the present embodiment, a thermistor type temperature sensor of a precision of ±0.5°C is used, and the temperature is monitored at intervals of 1 second.

The timer 250 provides a function of automatically turning off after driving the light irradiation part 110 for a preset time. The timer 250 may be implemented as a software implementation of the control part 230 or a separate hardware timer circuit. In the present embodiment, the timer may be set in 4 steps of 30 seconds, 1 minute, 2 minutes, and 5 minutes, and when a user selects a specific time, the device is automatically terminated after lapse of the corresponding time.

The light amount adjusting part 260 provides a function in which the user can adjust a light amount of the light irradiation part 110 as necessary. The light amount is adjustable in 5 steps of 10 mW/cm², 25 mW/cm², 40 mW/cm², 55 mW/cm², and 70 mW/cm². Light amount adjustment is implemented in a manner of controlling current supplied to the LEDs, and precise light amount adjustment is possible through a PWM (Pulse Width Modulation) control manner.

The user interface 270 is composed of a power button, a timer setting button, a light amount adjustment button, and an LED indicator displaying a current state. The user may turn the power of the device on and off through this interface, set a timer time, and adjust the light amount. The LED indicator visually displays information such as a power state, a battery remaining amount, and a currently set timer and light amount.

These additional components are integrated inside the main body 100, so they are not visible in appearance, but greatly improve performance, safety, and convenience of use of the device. Particularly, a combination of the heat dissipation structure 220 and the temperature sensor 240 effectively solves an overheating problem that may occur upon use of the high output LEDs, and the timer 250 and the light amount adjusting part 260 allow the user to custom-set the device to suit his/her skin state and need.

FIG. 3 is a view showing a structure for each type of a light irradiation device for skin care according to various embodiments of the present disclosure.

### Embodiment 3: Handy Type Light Irradiation Device

As shown in FIG. 3, a handy type light irradiation device 310, which is an embodiment of the present disclosure, includes a main body 311, a light irradiation part 312, a handle 313, and a window 314.

The main body 311 has a circular shape of a diameter of about 5 cm to 10 cm, and is manufactured in an ergonomic design that the user can easily grip by hand. An outer material of the main body 311 is composed of ABS resin having excellent heat resistance and durability, and a heat dissipation structure is included inside. The heat dissipation structure includes a heat sink and a heat dissipation fin manufactured of an aluminum alloy, to effectively discharge heat generated during operation of the high output LEDs.

The light irradiation part 312 includes a total of 12 LEDs, and a wavelength of light generated by each LED may be 380 nm to 570 nm, and as an example, blue light in a wavelength band of 400 nm to 480 nm is generated. The LEDs are designed such that 4 are arranged in a center part and 8 are arranged in a peripheral part to enable uniform light irradiation. A light amount of the light irradiation part 312 is set to 40 mW/cm², to provide an output about 3 times or more higher than a conventional low output LED beautifying device.

In this case, the light amount may be measured by contacting the transparent window 120 with a PM100D Energy Sensor & Power Meter of Thorlabs. In this case, it may be measured closely. By measuring the light amount by contacting the window, the light amount reaching the skin to be irradiated may be measured.

The skin to be irradiated may include a face, a neck, and a body, and in the case of the face, it may be forehead, cheek, and jaw (including jawline) part except for eyes. In this case, a head part (not shown) is connected to the main body 100, and may be provided according to a form and a size of the skin to be irradiated. As an example, in the case where the skin to be irradiated is a cheek or a jawline of a face, a head may be a form which may be a head in which a hole corresponding to a size of the facial cheek or the jawline is formed, and in the case where the skin to be irradiated is lips, it may be a head having a hole of a size corresponding to a lip size. In this case, the head may be attached and detached by a magnet, and the like.

In this case, in the case of a head with a large hole, its height may be large to increase a light irradiation area, and in the case of a head with a small hole, its height may be small to increase light focus.

A light amount of the light irradiation part may be adjusted in at least one or more light amount modes. As an example, the light irradiation part may be driven in a first light amount mode (10 to 30 mW/cm²), a second light amount mode (30 to 50 mW/cm²), and a third light amount mode (50 to 70 mW/cm²).

As an example, in the case of being used for skin to be irradiated of a relatively large area such as a cheek, a head with a large size of a hole may be connected to the main body. Further, in this case, it may be used in a mode in which a magnitude of a light amount is relatively large (second light amount mode or third light amount mode), and in the case of irradiating to lips, and the like, it may be used in a mode in which a magnitude of a light amount is relatively small (the first light amount mode) by connecting a head with a small size of a hole.

The handle 313 is connected to a side surface or an upper end of the main body 311, and is designed so that the user can comfortably grip and operate the device. The handle 313 is finished with a soft touch material to which a non-slip treatment is applied, to provide a stable grip feeling.

The window 314 is manufactured of a circular transparent acrylic material of a diameter of about 4 cm to 8 cm, and is located between the light irradiation part 312 and the skin so that light generated from the LEDs can be effectively delivered to the skin. A plurality of small holes (diameter of about 1 mm) are uniformly distributed in the window 314, to promote heat discharge along with light delivery.

The handy type light irradiation device 310 is designed to be used for a short time of at most within 15 minutes, preferably 1 to 5 minutes, and there is an advantage that selective concentrated light irradiation to a site desired by the user is possible.

### Embodiment 4: Mask Type Light Irradiation Device

A mask type light irradiation device 320, which is another embodiment of the present disclosure, includes a main body 321, a light irradiation part 322, a fixing band 323, and a facial shape curved surface 324.

The main body 321 is designed in a curved structure tailored to a facial form, and is manufactured of a lightweight plastic material to minimize a weight burden upon wearing. A heat dissipation structure and a control circuit are built in inside the main body 321.

The light irradiation part 322 includes a total of 120 LEDs capable of covering the entire face. The LEDs generate light in a wavelength band of 400 nm to 480 nm, and a light amount is set to 30 mW/cm². Although it is a somewhat lower light amount than the handy type, there is an advantage that light can be uniformly irradiated to the entire face. The LEDs are intensively arranged in sites where wrinkles are easily generated, such as around a forehead, cheeks, a jaw, and a nose.

The fixing band 323 is a detachable elastic band, and serves to stably fix the mask type device to the face. The fixing band 323 is designed to be adjustable to suit a head size of the user.

The facial shape curved surface 324 is a curved surface designed to suit a general facial form, and is located at an inner side of the main body 321. This curved surface optimizes a distance between the LEDs and the skin to enable effective light irradiation, and at the same time, serves to improve a wearing feeling.

For the mask type device 320, use within at most 30 minutes is recommended, and a heat sensing sensor and an automatic temperature adjustment function are mounted inside, and when an internal temperature of the mask exceeds 40 °C during use, an appropriate temperature is maintained by automatically adjusting output of the LEDs.

### Embodiment 5: Cradle Type Light Irradiation Device

A cradle type light irradiation device 330, which is another embodiment of the present disclosure, includes a main body 331, a support 332, an angle adjusting part 333, a light irradiation part 334, and a height adjusting part 335.

The main body 331 is a structure in a stand form that can be installed on a table or a floor, and a control circuit and a cooling system are built in inside. The main body 331 is manufactured of a combination of solid metal and plastic materials to provide a stable supporting force.

The support 332 is a structure stably supporting the main body 331 on the floor or the table, and ensures stability of the device by including a wide bottom surface and a non-slip pad.

The angle adjusting part 333 provides a function of being capable of adjusting an angle of the light irradiation part 334 within a range of 0° to 90°. The user may set the light irradiation part at an optimum angle according to his/her posture or a skin care site.

The light irradiation part 334 is a large LED panel, and a total of 200 LEDs are uniformly arranged. The LEDs have a function of being capable of selectively using various wavelength bands between 380 nm to 570 nm, so that a wavelength can be adjusted to suit a skin state or a purpose.

The height adjusting part 335 is connected to the support 332 to provide a function of being capable of adjusting a height of the light irradiation part 334. The height is adjustable within a range of 50 cm to 150 cm, so that the user may conveniently use even in a sitting posture or a lying posture.

A light amount of the cradle type device 330 is adjustable up to at most 70 mW/cm², and the user may adjust the light amount in units of 10 mW/cm² according to a skin state or a purpose. Such a high light amount is supported through a powerful cooling system, and a high-performance heat sink and a cooling fan are installed inside the device to effectively control generated heat.

For the cradle type device 330, use within at most 1 hour is recommended, and for convenience of the user, various functions such as a timer (5 minutes/10 minutes/15 minutes/20 minutes/30 minutes/45 minutes/60 minutes), automatic power cutoff, and LED panel angle adjustment are provided.

### Embodiment 6: Combined Use of Light Irradiation Device and Photoreactive Material

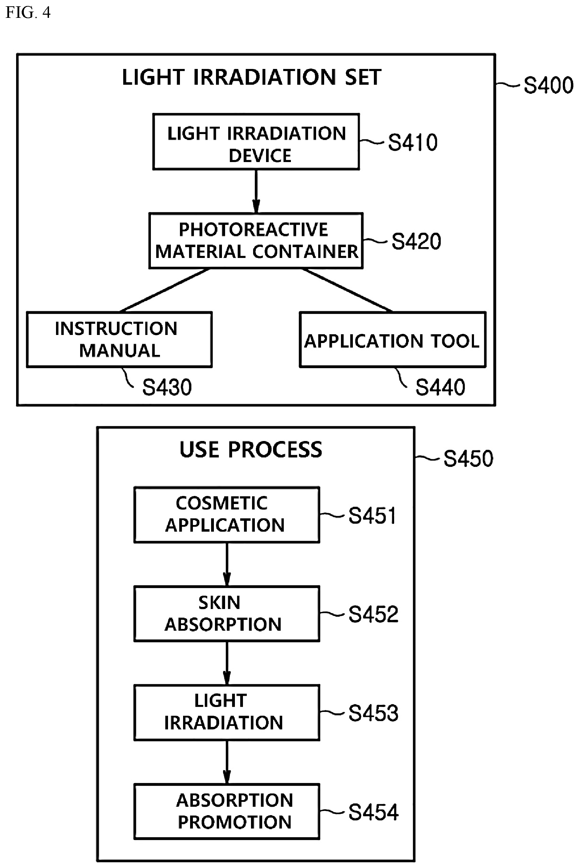

FIG. 4 is a view showing a set configuration and a use process comprising a light irradiation device and a photoreactive material according to an embodiment of the present invention.

As shown in FIG. 4, a light irradiation set 400 of the present disclosure includes a light irradiation device 410, a photoreactive material container 420, and an instruction manual 430, and in an embodiment, may further include an application tool 440. However, the application tool 440 may be excluded from the set configuration. Also, a use process 450 of the light irradiation set includes steps of cosmetic application 451, skin absorption 452, light irradiation 453, and absorption promotion 454.

The light irradiation device 410 may be a light irradiation device of any form (handy type, mask type, cradle type) described in FIG. 1 to FIG. 3, and in the present embodiment, a handy type light irradiation device is used as an example. The light irradiation device 410 is configured to irradiate light in a wavelength band of 380 nm to 570 nm, preferably 400 nm to 480 nm, with a light amount of 10 to 70 mW/cm².

The photoreactive material container 420 is a container containing a cosmetic composition comprising a photoreactive material. The photoreactive material container 420 is manufactured of a material having excellent light-shielding property and airtightness in consideration of characteristics of contents, and a capacity of 30 ml to 50 ml is common. The photoreactive material container 420 may be designed in a pump type or a tube type so that the user can easily take out the contents. Alternatively, it may be provided in a form of a disposable ampoule to be configured to be used immediately by opening upon use.

The photoreactive material is a component that is activated in response to light in a wavelength band of 400 nm to 480 nm, and performs a role of promoting a biochemical reaction within skin tissue by absorbing light energy of this wavelength band. As a representative photoreactive material used in the present embodiment, it may be riboflavin, a salt thereof, or a derivative thereof.

In an embodiment, the photoreactive material may be the photoreactive composition according to an embodiment of the present disclosure described above.

The instruction manual 430 is a document explaining in detail an effective and safe method of use of the light irradiation set 400. In the instruction manual 430, an application method of the cosmetic composition, a method of use of the light irradiation device, a recommended use time, precautions, and the like are included.

The application tool 440 is a tool for effectively applying a cosmetic composition containing a photoreactive material to skin, and is provided in a form of a spatula of a silicone material, a soft brush, or a sponge applicator. The application tool 440 is designed so that the cosmetic composition can be applied in a uniform thickness, so it helps in maximizing a light irradiation effect.

The cosmetic application 451, which is a first step of the use process 450, is a process of applying a cosmetic composition in which a photoreactive material is included to skin which is cleanly washed, thinly and uniformly by using the application tool 440. Upon application, in the case of a facial part, it is recommended to gently spread as if drawing a circle toward an outside starting from a T-zone.

The skin absorption 452, which is a second step, is a process of waiting for 1 to 2 minutes so that the applied cosmetic composition is sufficiently absorbed into the skin. In this process, the photoreactive material becomes uniformly distributed in a skin epidermis layer, so that a subsequent light irradiation effect can be maximized.

The light irradiation 453, which is a third step, is a process of irradiating light for 3 minutes to a site where a cosmetic is applied by using the light irradiation device 410. Upon light irradiation, a distance between the skin and the light irradiation device is maintained at 2 mm to 30 mm, and irradiation may be performed by concentrating on a specific site or a wide area may be uniformly irradiated.

The absorption promotion 454, which is a last step, is a process of absorbing remaining cosmetic components after light irradiation by lightly tapping on the skin. In this process, the photoreactive material activated by light irradiation penetrates deep into the skin to promote collagen and elastin generation, and maximizes a skin elasticity improvement effect and a wrinkle relief effect.

As such, the light irradiation set of the present invention using a light irradiation device and a photoreactive material together provides an improved synergy effect than sole use of each component, and provides an effective and convenient home care solution to a user.

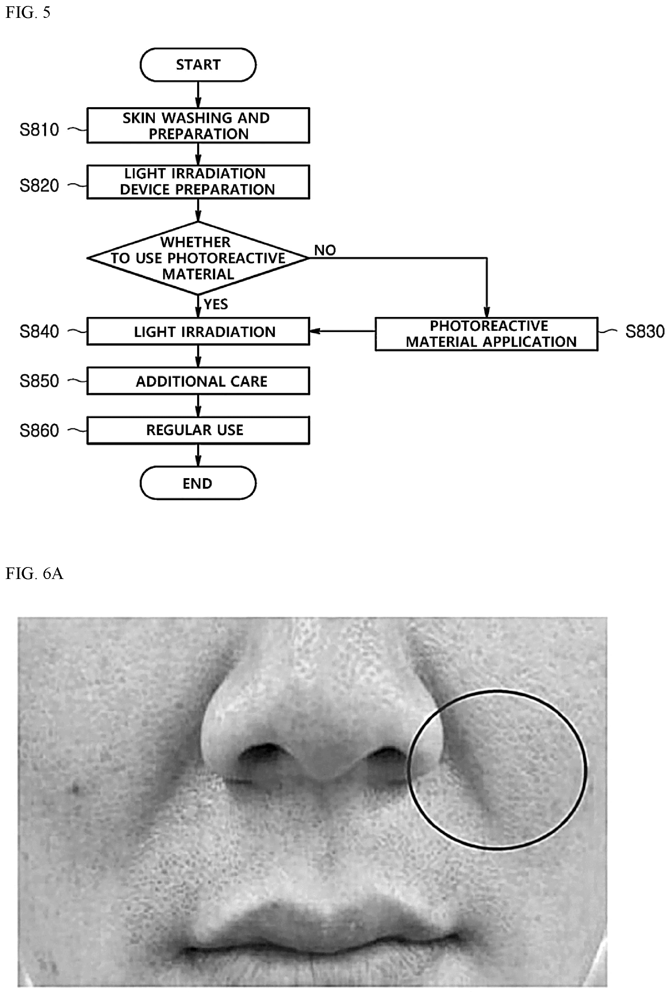

FIG. 5 is a flowchart of a skin care method using a light irradiation device according to an embodiment of the present disclosure. Recommended procedures for effectively and safely using a light irradiation device according to an embodiment of the present disclosure are shown step by step.

First, in a skin washing and preparation step (S810), skin is cleanly washed and dried to be prepared in an optimum state for light irradiation. In this step, makeup, skin secretions, dead skin cells, and the like are removed so that blue light can effectively act on the skin. After washing with lukewarm water, it is good to remove moisture with a soft towel, and in this case, it is dried in a manner of gently tapping so as not to give irritation to the skin.

Next, in a light irradiation device preparation step (S820), a form (handy type, mask type, cradle type) suitable for a need among the light irradiation devices of the present invention described in FIG. 1 to FIG. 3 is selected and the power is turned on. A light amount (10 mW/cm² to 70 mW/cm²), a wavelength band (within 380 nm to 570 nm), a timer time, and the like are set according to the need of the user and a care site. In the case of the handy type, within 15 minutes, in the case of the mask type, within 30 minutes, and in the case of the cradle type, a use time within 1 hour is recommended.

Optionally, in the case of using a photoreactive material, a photoreactive material application step (S830) is proceeded. In this step, a cosmetic composition comprising a photoreactive material is uniformly applied to the skin and is absorbed into the skin for 1 to 2 minutes. The photoreactive material may obtain a more excellent effect in a shorter time by amplifying an effect of blue light, but it is not an essential step.

Next, in a light irradiation step (S840), blue light is irradiated to the skin by using the light irradiation device of the present invention. In the case of a handy type device, a distance between the skin and the device is maintained at 2 to 30 mm and blue light is directly irradiated to a site to be cared for. In the case of a mask type, it is worn by being closely attached to the face, and in the case of a cradle type, light irradiation is made to be uniformly performed by adjusting an appropriate distance and an angle. In this case, components such as the control part 230, the temperature sensor 240, and the timer 250 automatically operate so that safe and effective light irradiation is performed.

After light irradiation, it moves on to an additional care step (S850). In this step, a moisturizer or a cosmetic having a soothing effect may be used to soothe and moisturize the skin which has become slightly warm due to light irradiation. Particularly, in the case of a user whose skin is dry, this step is important.

Finally, a regular use step (S860) recommends using the light irradiation device of the present invention regularly 2 to 3 times a week for an optimum effect. A light irradiation device according to an embodiment of the present disclosure provides a cumulative effect upon continuous use along with an immediate effect. Particularly, a nasolabial fold improvement effect and a keratin density increase effect may become more distinct through regular use.

The steps above show a basic skin care method using the light irradiation device of the present disclosure, and details of some steps may be adjusted according to a skin state, an age, a gender, a care purpose, and the like of the user. Also, the photoreactive material application step (S830) is an optional step, and this step may be omitted and it may move directly to the light irradiation step (S840).

A skin care method using the light irradiation device of the present disclosure has an advantage that a more distinct effect can be obtained in a shorter time compared to a method using a conventional low output LED beautifying device. Particularly, it is designed so that effective skin care is possible within a short time in accordance with a life pattern of busy modern people.

Another embodiment of the present disclosure may provide a skin care kit comprising: the composition described above; and a light irradiation device comprising a light irradiation part.

In an embodiment, the light irradiation device may include: a main body forming a housing in which a light irradiation part is installed; the light irradiation part provided in the main body and irradiating light in a wavelength band of 380 nm to 570 nm; and a power supply part supplying power to the light irradiation part.

In an embodiment, the light irradiation part may be for irradiating visible light of a blue light series. Specifically, a wavelength range of the irradiated light may be 380 nm or more, 400 nm or more, 420 nm or more, 440 nm or more, 460 nm or more, 480 nm or more, 500 nm or more, 520 nm or more, 540 nm or more, or 560 nm or more, and may be 570 nm or less, 550 nm or less, 530 nm or less, 510 nm or less, 490 nm or less, 470 nm or less, 450 nm or less, 430 nm or less, 410 nm or less, or 390 nm or less.

In an embodiment, the light irradiation part may comprise a plurality of LEDs. In an embodiment, the main body may comprise a transparent or translucent window located between the light irradiation part and the skin.

In an embodiment, a light amount of the light irradiation part may be 1 to 100 mW/cm². Specifically, the light amount may be 1 mW/cm² or more, 10 mW/cm² or more, 20 mW/cm² or more, 30 mW/cm² or more, 40 mW/cm² or more, 50 mW/cm² or more, 60 mW/cm² or more, 70 mW/cm² or more, 80 mW/cm² or more, or 90 mW/cm² or more, and may be 100 mW/cm² or less, 90 mW/cm² or less, 80 mW/cm² or less, 70 mW/cm² or less, 60 mW/cm² or less, 50 mW/cm² or less, 40 mW/cm² or less, 30 mW/cm² or less, 20 mW/cm² or less, or 10 mW/cm² or less.

In an embodiment, an energy range irradiated by the light irradiation part may be 1 to 10 J.

In an embodiment, the main body may comprise a heat dissipation structure for discharging heat generated from the light irradiation part. The heat dissipation structure is a structure for effectively discharging heat generated during operation of the light irradiation part, and may be installed inside the main body.

In an embodiment, the light irradiation device may further comprise a temperature sensor measuring a temperature of the light irradiation part and a control part limiting operation of the light irradiation part when the temperature measured by the temperature sensor exceeds a preset temperature. In an embodiment, the temperature of the light irradiation part may be 30°C to 60°C.

In an embodiment, the light irradiation device may further comprise a timer automatically turning off after driving the light irradiation part for a preset time. For example, the preset time may be 1 to 60 minutes, but the set time may be appropriately adjusted according to a form of the light irradiation device, a light amount of the light irradiation part, or a method of use.

As an embodiment, the light irradiation time may be 1 to 60 minutes. Specifically, the light irradiation time may be 1 minute or more, 5 minutes or more, 10 minutes or more, 15 minutes or more, 20 minutes or more, 25 minutes or more, 30 minutes or more, 35 minutes or more, 40 minutes or more, 45 minutes or more, 50 minutes or more, or 55 minutes or more, and may be 60 minutes or less, 55 minutes or less, 50 minutes or less, 45 minutes or less, 40 minutes or less, 35 minutes or less, 30 minutes or less, 25 minutes or less, 20 minutes or less, 15 minutes or less, 10 minutes or less, or 5 minutes or less.

In an embodiment, a distance between the light irradiation part and the skin may be 0.1 to 30 cm, and the distance may be appropriately adjusted according to a form of the light irradiation device, a light amount of the light irradiation part, or a method of use. Specifically, the distance between the light irradiation part and the skin may be 0.1 cm or more, 1 cm or more, 2 cm or more, 3 cm or more, 4 cm or more, 5 cm or more, 6 cm or more, 7 cm or more, 8 cm or more, 9 cm or more, 10 cm or more, 15 cm or more, 20 cm or more, or 25 cm or more, and may be 30 cm or less, 25 cm or less, 20 cm or less, 15 cm or less, 10 cm or less, 9 cm or less, 8 cm or less, 7 cm or less, 6 cm or less, 5 cm or less, 4 cm or less, 3 cm or less, 2 cm or less, or 1 cm or less. When the light irradiation distance becomes too far, a light amount reaching the skin is small, so an effect may decrease.

In an embodiment, the light irradiation device may be any one of a handy type, a mask type, or a cradle type.

In an embodiment, the skin care kit may further comprise an eye protection device.

In an embodiment, the skin care kit may further comprise an instruction manual in which an application method of the composition, a method of use of the light irradiation device, a recommended use time, precautions, and the like are included. For example, the manual may identically comprise those described in a skin beautifying method to be described later.

In an embodiment, the instruction manual may comprise a skin care method comprising the steps of: applying the composition to skin; and irradiating light in a wavelength band of 380 nm to 570 nm to the skin to which the composition is applied, by using the light irradiation device.

As an embodiment, a light irradiation time of the step of irradiating the light may be 1 to 60 minutes. Specifically, the light irradiation time may be within 1 hour in the case where the light irradiation device is a cradle type device, within 30 minutes in the case of a device in a mask form, and within 15 minutes in the case of a handy type device.

An embodiment of the present disclosure may provide a skin beautifying method comprising the steps of: applying a photoreactive composition to skin; and irradiating light in a wavelength band of 380 nm to 570 nm to the skin to which the composition is applied.

In an embodiment, the method may be one satisfying one or more of: a light amount of the irradiated light being 1 to 100 mW/cm²; a light irradiation distance with respect to the skin being 0.1 to 30 cm; and a light irradiation time being 1 to 60 minutes, and specific details for each light irradiation condition may be identically applied as described above.

In an embodiment, the method may be a method for prevention of skin wrinkle generation or wrinkle improvement, or prevention of skin elasticity decrease or elasticity improvement.

As an embodiment, the step of applying the photoreactive composition to skin may comprise applying the composition directly by hand or applying by using various application means such as a spatula, a brush, and a puff.

In an embodiment, the step of irradiating the light may be irradiating light after 1 second to 1 hour after applying the photoreactive composition to skin. Specifically, the irradiation of the light may be irradiating light after lapse of 1 second or more, 30 seconds or more, 1 minute or more, 2 minutes or more, 3 minutes or more, 4 minutes or more, 5 minutes or more, 6 minutes or more, 7 minutes or more, 8 minutes or more, 9 minutes or more, 10 minutes or more, 20 minutes or more, 30 minutes or more, 40 minutes or more, or 50 minutes or more, and at the same time, 60 minutes or less, 50 minutes or less, 40 minutes or less, 30 minutes or less, 20 minutes or less, 10 minutes or less, 9 minutes or less, 8 minutes or less, 7 minutes or less, 6 minutes or less, 5 minutes or less, 4 minutes or less, 3 minutes or less, 2 minutes or less, or 1 minute or less, after applying the photoreactive composition to skin. When the time interval exceeds 1 hour, the composition applied to the skin is decomposed by other light such as a fluorescent lamp, so an effect may decrease.

Also, in an embodiment, a performance cycle of the skin beautifying method may be daily, every other day, once a week, twice a week, three times a week, five times a week, six times a week, once a month, twice a month, three times a month, or four times a month, and the like. The performance cycle may be regular or irregular, and may be adjusted according to a skin state or a purpose of use of the user.

Hereinafter, the present disclosure will be described more specifically through following examples.

### [Test Example 1] Confirmation of Immediacy of Nasolabial Fold Improvement Effect

To confirm the immediacy of a nasolabial fold improvement effect according to an embodiment according to the present disclosure, the following experiment was carried out.

First, an aqueous solution comprising 0.05 wt% of riboflavin 5'-phosphate based on a total weight of the composition and 40 wt% of pentylene glycol, which is an absorption promoter, was applied in an amount of 0.5 ml to a face of a subject having normal skin, and then absorbed for 1 minute. Thereafter, blue light (450 to 455 nm) was irradiated for 2 minutes (Example 1).

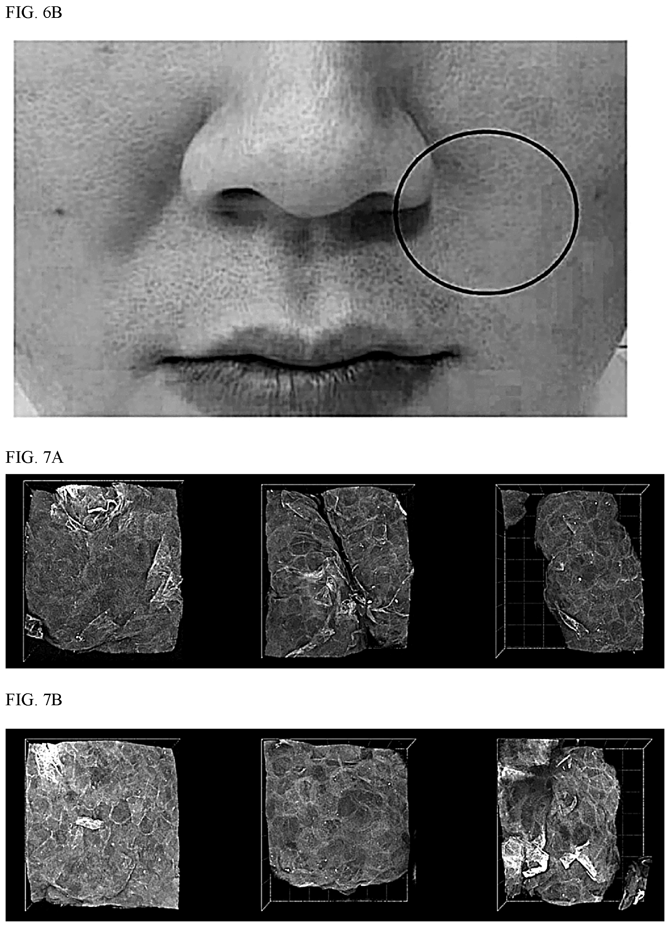

FIG. 6A is a facial photograph of a subject (untreated group) in a state before treatment with the composition and light irradiation, and nasolabial folds (indicated by a red circle) are clearly observed. FIG. 6B is a facial photograph of a subject taken within 10 minutes after application of the composition and blue light irradiation according to the Example 1. As can be seen in FIG. 6B, it can be confirmed that a depth of the nasolabial folds significantly decreased and a skin surface became smooth after treatment. This means that nasolabial folds can be improved in a short time by application of a composition according to the present disclosure and blue light irradiation.

### [Test Example 2] Confirmation of Durability of Nasolabial Fold Improvement Effect

To confirm the durability of a nasolabial fold improvement effect according to an embodiment according to the present disclosure, the following experiment was carried out.

First, an aqueous solution comprising 0.05 wt% of riboflavin 5'-phosphate based on a total weight of the composition and 40 wt% of pentylene glycol, which is an absorption promoter, was applied in an amount of 0.5 ml to a face of a subject having normal skin, and then absorbed for 1 minute. Thereafter, blue light (450 to 455 nm) was irradiated for 2 minutes (Example 1).

Then, by using a PRIMOS premium (LMI Technologie) device, a surface roughness Ra (Roughness Average) as a parameter of a nasolabial fold improvement degree was measured immediately after the light irradiation and at a time when 1 day (24 hours) elapsed (N=5), and its results are shown in Table 1. In this case, a measurement value (Ra) of an untreated group means a result value measured on the face of the subject in a state before treatment with the composition and light irradiation, and a reduction value of Example 1 compared to the untreated group is a degree of reduction of a wrinkle measurement value upon treatment of Example 1 compared to the measured measurement value of the untreated group expressed as a percentage.

**TABLE 1**

| Wrinkles | Before use | Immediately after use | After 1 day |
|---|---|---|---|
| Measurement value of untreated group (Ra) | 18.358 | 17.525 | 16.651 |
| Reduction value of Example 1 compared to untreated group | - | -4.54% | -9.30% |

From the results above, it can be confirmed that a skin wrinkle improvement effect by application of a composition according to the present disclosure and blue light irradiation is maintained for at least 24 hours or more.

### [Test Example 3] Confirmation of Skin Tissue Density Increase 1

A synergistic effect by application of a composition according to an embodiment according to the present disclosure and blue light irradiation was confirmed through a density change analysis of skin tissue.

First, after treating 20 µl of an aqueous solution comprising 0.05 wt% of riboflavin 5'-phosphate based on a total weight of the composition and 40 wt% of pentylene glycol, which is an absorption promoter, to cadaver skin (Type 2, Epidermis&Dermis, manufacturer: Biohead) (3x3 cm²), absorption was induced for 3 minutes. Thereafter, after irradiating blue light (450 to 455 nm) for 2 minutes, formalin was treated to fix so that there is no additional reaction. (Example 1)

As comparative examples, Comparative Example 1 treated only with a riboflavin 5'-phosphate aqueous solution in the same manner as above and Comparative Example 2 treated only with blue light irradiation were prepared, and as a control group, an untreated group treated with neither riboflavin 5'-phosphate nor blue light was prepared.

Then, a keratin density of the Example 1, the Comparative Examples 1 and 2, and the control group prepared as above was analyzed and confirmed with a fluorescence image through a UV/VIS confocal laser scanning microscope (Carl Zeiss).

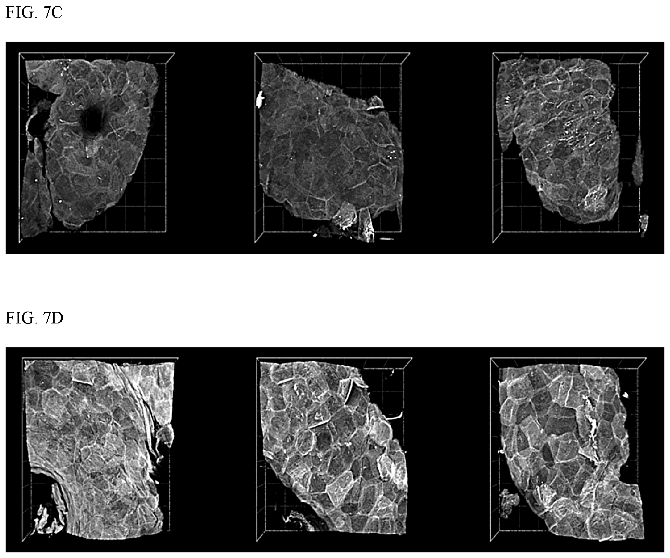

As a result, in the Comparative Example 1 (riboflavin 5'-phosphate alone-treated group, FIG. 7B) and the Comparative Example 2 (blue light alone-treated group, FIG. 7C), a density (green fluorescence) similar to that of the control group (untreated group, FIG. 7A) was shown, but in the Example 1 (riboflavin 5'-phosphate + blue light treated group, FIG. 7D) according to the present disclosure, it can be confirmed that the density (green fluorescence) significantly increases. This means that a density of protein providing structural stability and elasticity of the skin increased due to a synergistic effect of riboflavin 5'-phosphate and blue light irradiation according to an embodiment of the present disclosure, and through this, an excellent improvement effect of skin wrinkles can be provided.

### [Test Example 4] Confirmation of Skin Tissue Density Increase 2

A density change of skin tissue according to addition of an absorption promoter of a composition according to an embodiment according to the present disclosure was confirmed through an experiment below.

First, after treating 20 µl of an aqueous solution comprising 0.05 wt% of riboflavin 5'-phosphate based on a total weight of the composition and 40 wt% of pentylene glycol, which is an absorption promoter, to cadaver skin (Type 2, Epidermis&Dermis, manufacturer: Biohead) (2x2 cm²), absorption was induced for 10 minutes. After blue light (450 to 455 nm) was irradiated (Example 1), tensile strength was measured by using a universal testing machine (QMESYS) under blue light (450 to 455 nm).

As another embodiment, after treating 20 µl of an aqueous solution in which riboflavin 5'-phosphate is comprised in an amount of 0.05 wt% based on a total weight of the composition to cadaver skin (Type 2, Epidermis&Dermis, manufacturer: Biohead) (2x2 cm²), absorption was induced for 10 minutes. Thereafter, tensile strength was measured by using a universal testing machine (QMESYS) under blue light (450 to 455 nm). (Example 2)

**TABLE 2**

| | Untreated group | Example 1 | Example 2 |
|---|---|---|---|
| Tensile strength | 0.86 | 2.54 | 0.90 |

| | | | |
|---|---|---|---|
| (Unit: Kgf) | | | |

As a result, as shown in the Table 2, in the case of the Example 1 to which the absorption promoter was added, the tensile strength increased 2.95 times compared to the untreated group. This means that absorption of the riboflavin 5'-phosphate into the skin was promoted by the absorption promoter, so that a skin tissue density increased. In the case of the Example 2, almost no difference from the untreated group appeared, and this is because the riboflavin 5'-phosphate was hardly absorbed into the skin, so that it failed to increase the skin tissue density.

The present disclosure may provide following embodiments as an embodiment.

### [First Embodiment]

A photoreactive composition comprising riboflavin 5'-phosphate or a salt thereof as an active ingredient, the composition further comprising an absorption promoter of the riboflavin 5'-phosphate or the salt thereof, and for being used in combination with light irradiation of a wavelength of 380 to 570 nm.

### [Second Embodiment]

The photoreactive composition according to the first embodiment, wherein the composition comprises the riboflavin 5'-phosphate or the salt thereof in an amount of 0.0001 to 10 wt% based on a total weight of the composition.

### [Third Embodiment]

The photoreactive composition according to the first or second embodiment, wherein the absorption promoter is one or more selected from the group consisting of propylene glycol, pentylene glycol, hexanediol, bis-ethoxydiglycol cyclohexane 1,4-dicarboxylate, diethoxyethyl succinate, dimethyl isosorbide, polyglyceryl-6 caprylate, and polyglyceryl-4 caprate.

### [Fourth Embodiment]

The photoreactive composition according to any one of the first to third embodiments, wherein the composition comprises the absorption promoter in an amount of 1 to 40 wt% based on a total weight of the composition.

### [Fifth Embodiment]

The photoreactive composition according to any one of the first to fourth embodiments, wherein the composition is for being used in a skin beautifying method comprising one or more of: a light amount of the irradiated light being 1 to 100 mW/cm²; a light irradiation distance with respect to the skin being 0.1 to 30 cm; and a light irradiation time being 1 to 60 minutes.

### [Sixth Embodiment]

The photoreactive composition according to any one of the first to fifth embodiments, wherein the composition is a cosmetic composition.

### [Seventh Embodiment]

The photoreactive composition according to any one of the first to sixth embodiments, wherein the composition is for prevention of skin wrinkle generation or wrinkle improvement, or prevention of skin elasticity decrease or elasticity improvement.

### [Eighth Embodiment]

The photoreactive composition according to any one of the first to seventh embodiments, wherein the composition is for improvement of nasolabial folds.

### [Ninth Embodiment]

The photoreactive composition according to any one of the first to eighth embodiments, wherein the composition is a pharmaceutical composition.

### [Tenth Embodiment]

The photoreactive composition according to any one of the first to ninth embodiments, further comprising a manual in which a skin care method comprising: applying the cosmetic composition to skin; and irradiating light in a wavelength band of 380 nm to 570 nm to the skin to which the composition is applied, by using a light irradiation device, is described.

### [Eleventh Embodiment]

A skin care kit comprising: the photoreactive composition according to any one of the first to tenth embodiments; and a light irradiation device comprising a light irradiation part.

### [Twelfth Embodiment]

The skin care kit according to the eleventh embodiment, wherein the light irradiation device comprises: a main body forming a housing in which the light irradiation part is installed; the light irradiation part provided in the main body and irradiating light in a wavelength band of 380 nm to 570 nm; and a power supply part configured to supply power to the light irradiation part.

### [Thirteenth Embodiment]

The skin care kit according to the eleventh or twelfth embodiment, wherein the light irradiation part comprises a plurality of LEDs.

### [Fourteenth Embodiment]

The skin care kit according to any one of the eleventh to thirteenth embodiments, wherein a light amount of the light irradiation part is 1 to 100 mW/cm².

### [Fifteenth Embodiment]

The skin care kit according to any one of the eleventh to fourteenth embodiments, wherein the light irradiation device is any one of a handy type, a mask type, or a cradle type.

### [Sixteenth Embodiment]

The skin care kit according to any one of the eleventh to fifteenth embodiments, further comprising a manual in which a skin care method comprising: applying the composition to skin; and irradiating light in a wavelength band of 380 nm to 570 nm to the skin to which the composition is applied, by using the light irradiation device, is described.

### [Seventeenth Embodiment]

A skin beautifying method comprising: applying a photoreactive composition according to any one of the first to tenth embodiments to skin; and irradiating light in a wavelength band of 380 nm to 570 nm to the skin to which the composition is applied.

### [Eighteenth Embodiment]

The skin beautifying method according to the seventeenth embodiment, wherein the method satisfies one or more of: a light amount of the irradiated light being 1 to 100 mW/cm²; a light irradiation distance with respect to the skin being 0.1 to 30 cm; and a light irradiation time being 1 to 60 minutes.

### [Nineteenth Embodiment]

The skin beautifying method according to the seventeenth or eighteenth embodiment, wherein the method is a method for prevention of skin wrinkle generation or wrinkle improvement, or prevention of skin elasticity decrease or elasticity improvement.

### [Twentieth Embodiment]

A light irradiation device for skin care, comprising: a main body forming a housing in which a light irradiation part is installed; the light irradiation part provided in the main body and irradiating light in a wavelength band of 380 nm to 570 nm; and a power supply part configured to supply power to the light irradiation part, wherein a light amount of the light irradiation part is 10 to 70 mW/cm².

### [Twenty-first Embodiment]

The light irradiation device according to the twentieth embodiment, wherein the light irradiation part satisfies one or more of: a wavelength being 400 nm to 480 nm; comprising a plurality of LEDs; a temperature being between 30 to 40°C upon use for 1 minute, and between 35 to 60°C upon use for 2 minutes; and an energy range irradiated being 3.2 J to 6.4 J.

### [Twenty-second Embodiment]

The light irradiation device according to the twentieth or twenty-first embodiment, wherein the main body satisfies one or more of: comprising a heat dissipation structure for discharging heat generated from the light irradiation part; and comprising a transparent or translucent window located between the light irradiation part and the skin.

### [Twenty-third Embodiment]

The light irradiation device according to any one of the twentieth to twenty-second embodiment, further comprising: a temperature sensor configured to measure a temperature of the light irradiation part; a control part configured to limit operation of the light irradiation part when the temperature measured by the temperature sensor exceeds a preset temperature; and a timer configured to automatically turn off after driving the light irradiation part for a preset time.

## Claims

1. A use of a photoreactive composition comprising riboflavin 5'-phosphate or a salt thereof as an active ingredient for being used in combination with light irradiation of a wavelength of 380 to 570 nm,
wherein the composition further comprises an absorption promoter of the riboflavin 5'-phosphate or the salt thereof, and preferably the absorption promoter is one or more selected from the group consisting of propylene glycol, pentylene glycol, hexanediol, bis-ethoxydiglycol cyclohexane 1,4-dicarboxylate, diethoxyethyl succinate, dimethyl isosorbide, polyglyceryl-6 caprylate, and polyglyceryl-4 caprate.

2. The use of claim 1, wherein the composition satisfies one or more of:
comprising the riboflavin 5'-phosphate or the salt thereof in an amount of 0.0001 to 10 wt% based on a total weight of the composition; and
comprising the absorption promoter in an amount of 1 to 40 wt% based on the total weight of the composition.

3. The use of claim 1 or 2, wherein the composition is for being used in a skin beautifying method comprising one or more of:
a light amount of the irradiated light being 1 to 100 mW/cm²;
a light irradiation distance with respect to the skin being 0.1 to 30 cm; and
a light irradiation time being 1 to 60 minutes.

4. The use of any one of claims 1 to 3, wherein the composition is a cosmetic composition, and preferably the cosmetic composition is for prevention of skin wrinkle generation or wrinkle improvement, prevention of skin elasticity decrease or elasticity improvement, or improvement of nasolabial folds.

5. A skin care kit (400), comprising:
a photoreactive composition comprising riboflavin 5'-phosphate or a salt thereof, and an absorption promoter of the riboflavin 5'-phosphate or the salt thereof, in which preferably the absorption promoter is one or more selected from the group consisting of propylene glycol, pentylene glycol, hexanediol, bis-ethoxydiglycol cyclohexane 1,4-dicarboxylate, diethoxyethyl succinate, dimethyl isosorbide, polyglyceryl-6 caprylate, and polyglyceryl-4 caprate; and
a light irradiation device (410) comprising a light irradiation part (110).

6. The kit (400) of claim 5, wherein the composition satisfies one or more of:
comprising the riboflavin 5'-phosphate or the salt thereof in an amount of 0.0001 to 10 wt% based on a total weight of the composition; and
comprising the absorption promoter in an amount of 1 to 40 wt% based on the total weight of the composition.

7. The kit (400) of claim 5 or 6, wherein the light irradiation device (410) comprises:
a main body (100) forming a housing in which the light irradiation part (110) is installed;
the light irradiation part (110) provided in the main body (100) and irradiating light in a wavelength band of 380 nm to 570 nm; and
a power supply part (210) configured to supply power to the light irradiation part (110).

8. The kit (400) of claim 7, wherein the light irradiation device (410) satisfies one or more of:
the light irradiation part (110) comprising a plurality of LEDs;
a light amount of the light irradiation part (110) being 1 to 100 mW/cm²; and
the light irradiation device (410) being any one of a handy type, a mask type, or a cradle type.

9. The kit (400) of any one of claims 5 to 8, further comprising a manual in which a skin care method comprising:
applying the composition to skin; and
irradiating light in a wavelength band of 380 nm to 570 nm to the skin to which the composition is applied, by using the light irradiation device, is described.

10. A skin beautifying method using the skin care kit of any one of claims 5 to 9, comprising:
applying the photoreactive composition to skin; and
irradiating light in a wavelength band of 380 nm to 570 nm to the skin to which the composition is applied, and preferably being a method for prevention of skin wrinkle generation or wrinkle improvement, or prevention of skin elasticity decrease or elasticity improvement.

11. The skin beautifying method of claim 10, wherein the method satisfies one or more of:
a light amount of the irradiated light being 1 to 100 mW/cm²;
a light irradiation distance with respect to the skin being 0.1 to 30 cm; and
a light irradiation time being 1 to 60 minutes.

12. A light irradiation device (410) for skin care, comprising:
a main body (100) forming a housing in which a light irradiation part (110) is installed;
the light irradiation part (110) provided in the main body (100) and irradiating light in a wavelength band of 380 nm to 570 nm; and
a power supply part (210) configured to supply power to the light irradiation part (110),
wherein a light amount of the light irradiation part (110) is 10 to 70 mW/cm².

13. The light irradiation device (410) of claim 12, wherein the light irradiation part (110) satisfies one or more of:
a wavelength being 400 nm to 480 nm;
comprising a plurality of LEDs;
a temperature being between 30 to 40°C upon use for 1 minute, and between 35 to 60°C upon use for 2 minutes; and
an energy range irradiated being 3.2 J to 6.4 J.

14. The light irradiation device (410) of claim 12, wherein the main body (100) satisfies one or more of:
comprising a heat dissipation structure for discharging heat generated from the light irradiation part (110); and
comprising a transparent or translucent window (120) located between the light irradiation part (110) and the skin.

15. The light irradiation device (410) of claim 12, further comprising:
a temperature sensor (240) configured to measure a temperature of the light irradiation part (110);
a control part (230) configured to limit operation of the light irradiation part (110) when the temperature measured by the temperature sensor (240) exceeds a preset temperature; and
a timer (250) configured to automatically turn off after driving the light irradiation part (110) for a preset time.
